(19) Europäisches Patentamt
European Patent Office
Office européen des brevets

(11) Veröffentlichungsnummer: **0 141 205**
**B1**

(12) **EUROPÄISCHE PATENTSCHRIFT**

(45) Veröffentlichungstag der Patentschrift: **06.06.90**

(21) Anmeldenummer: **84111054.7**

(22) Anmeldetag: **17.09.84**

(51) Int. Cl.$^5$: **C 07 D 249/08**, C 07 D 233/61, A 01 N 43/653, A 01 N 43/50

(54) **Hydroxyethylazolyl-oxim-Derivate.**

(30) Priorität: **26.09.83 DE 3334781**
**27.02.84 DE 3407005**

(43) Veröffentlichungstag der Anmeldung:
**15.05.85 Patentblatt 85/20**

(45) Bekanntmachung des Hinweises auf die Patenterteilung:
**06.06.90 Patentblatt 90/23**

(84) Benannte Vertragsstaaten:
**AT BE CH DE FR GB IT LI NL SE**

(56) Entgegenhaltungen:
**EP-A-0 065 107**
**EP-A-0 071 568**
**EP-A-0 078 594**
**EP-A-0 097 496**
**DE-A-3 018 866**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Krämer, Wolfgang, Dr.**
**Am Eckbusch 39**
**45 D-5600 Wuppertal 1 (DE)**
Erfinder: **Büchel, Karl Heinz, Prof. Dr.**
**Dabringhausener Strasse 42**
**D-5093 Burscheid (DE)**
Erfinder: **Holmwood, Graham, Dr.**
**Krutscheider Weg 105**
**D-5600 Wuppertal 11 (DE)**
Erfinder: **Reinecke, Paul, Dr.**
**Lessingstrasse 11**
**D-5090 Leverkusen 3 (DE)**
Erfinder: **Brandes, Wilhelm, Dr.**
**Eichendorffstrasse 3**
**D-5653 Leichlingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 141 205 B1**

## Beschreibung

Die vorliegende Erfindung betrifft neue Hydroxyethylazolyl-oxim-Derivate, mehrere Verfahren zu ihrer Herstellung und ihre Verwendung als Fungizide.

Es ist bereits bekannt geworden, daß bestimmte Hydroxyethylazol-Derivate, wie z.B. das 2-(4-Chlorphenoxymethyl)-3,3-dimethyl-1-(imidazol-1-yl)-butan-2-ol, gute fungizide Eigenschaften aufweisen (vgl. DE—A 3 018 866). Die Wirkung dieser Verbindungen ist jedoch bei niedrigen Aufwandmengen und Anwendungskonzentrationen nicht immer voll befriedigend.

Aus der EP—A 0 071 568 sind Azolylmethylcarbinol-Derivate mit fungiziden Eigenschaften bekannt. Es werden aber keine Stoffe offenbart, in denen am Carbinolkohlenstoffatom eine Oxim-Gruppe enthalten sein kann.

Ferner wurden in der EP—A 0 065 107 schon fungizid wirksame Azole mit einem Oxim-Rest im Molekül beschrieben. In diesen Verbindungen ist aber weder eine Azolyl-*methyl*-Gruppe noch ein Carbinolkohlenstoffatom vorhanden.

Es wurden nun neue Hydroxyethyloxim-Derivate der Formel

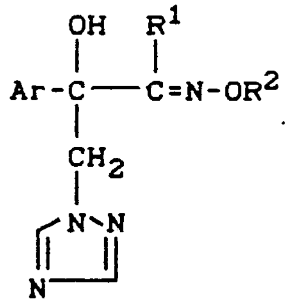

(I)

in welcher

Ar für Phenyl steht, das einfach oder mehrfach gleich oder verschieden substituiert sein kann durch Halogen, Alkyl mit bis 1 bis 4 Kohlenstoffatome, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, Alkoximinomethyl mit 1 bis 4 Kohlenstoffatomen in Alkoxyteil, Phenyl und Phenoxy,

$R^1$ für Wasserstoff oder geradkettiges oder verzweigtes Alkyl mit 1 bis 4 Kohlenstoffatomen steht und

$R^2$ für Wasserstoff, geradkettiges oder verzweigtes Alkyl mit 1 bis 4 Kohlenstoffatomen steht oder für Phenylalkyl mit 1 oder 2 Kohlenstoffatomen im Alkylteil steht, wobei der Phenylteil einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatom, Alkoximinomethyl mit 1 bis 4 Kohlenstoffatomen im Alkoxyteil, Phenyl und Phenoxy,

und deren Säureadditions-Salze und Metallsalz-Komplexe gefunden.

Weiterhin wurde gefunden, daß man die Hydroxyethylazolyloxim-Derivate der Formel (I) sowie deren Säureadditions-Salze und Metallsalz-Komplexe erhält, wenn man

a) Hydroxyethylazolyl-keto-Derivate der Formel

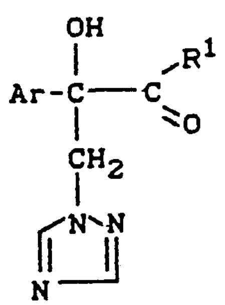

(II)

in welcher

Ar und $R^1$ die oben angegebene Bedeutung haben, wobei die Keto-Gruppe auch in Ketten- oder ringförmiger Acetal- bzw. Ketal-Struktur vorliegen kann,

mit Hydroxylamin-Derivaten der Formel

$$H_2N—O—R^2 \quad (III)$$

in welcher
$R^2$ die oben angegebene Bedeutung hat,
in Gegenwart eines Verdünnungsmittels umsetzt, und gegebenenfalls noch
b) nach Verfahren a) erhaltene Hydroxyethylazolyloxim-Derivate der Formel

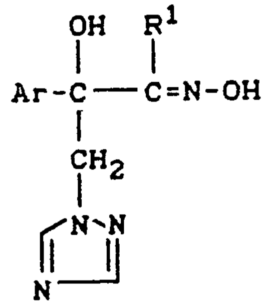
(Ia)

in welcher
Ar und $R^1$ die oben angegebene Bedeutung haben, mit Halogeniden der Formel

$$Hal—R^3 \quad (IV)$$

in welcher
Hal für Chlor, Brom oder Jod steht und
$R^3$ für die Bedeutungen von $R^2$, außer für Wasserstoff, steht,
in Gegenwart eines Verdünnungsmittels und gegebenenfalls in Gegenwart einer Base umsetzt; oder
c) Oxirane der Formel

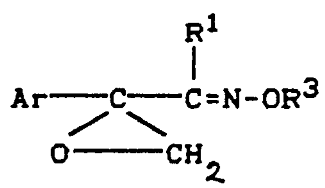
(V)

in welcher
Ar, $R^1$ und $R^2$ die oben angegebene Bedeutung haben,
mit Azolen der Formel

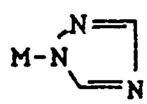
(VI)

in welcher
M für Wasserstoff oder ein Alkalimetall steht,
in Gegenwart eines Verdünnungsmittels und gegebenenfalls in Gegenwart einer Base umsetzt, und gegebenenfalls noch an die so erhaltenen Verbindungen der Formel (I) anschließend eine Säure oder ein Metallsalz addiert.

Die Verbindungen der Formel (I) können in zwei geometrischen Isomeren (syn- und anti-Form) vorliegen; vorwiegend fallen sie als Gemische unterschiedlicher Zusammensetzung beider Formen an.

Die erfindungsgemäßen Stoffe der Formel (I) sowie deren Säureadditions-Salze und Metallsalz-Komplexe zeigen überraschenderweise eine bessere fungizide Wirkung als die aus dem Stand der Technik bekannten Hydroxyethylazol-Derivate.

Die erfindungsgemäßen Wirkstoffe stellen somit eine Bereicherung der Technik dar.

Die erfindungsgemäßen Hydroxyethylazolyl-oxim-Derivate sin durch die Formel (I) allgemein definiert.

Bevorzugt sind diejenigen Verbindungen der Formel (I), in denen

Ar für gegebenenfalls einfach bis dreifach, gleich oder verschieden durch Fluor, Chlor, Methyl, Trifluormethyl, Methoximinomethyl, Ethoxyiminomethyl, Phenyl und/oder Phenoxy substituiertes Phenyl steht,

$R^1$ für Wasserstoff oder geradkettiges Alkyl mit 1 bis 4 Kohlenstoffatomen steht und

$R^2$ für Wasserstoff, geradkettiges Alkyl mit 1 bis 4 Kohlenstoffatomen oder für gegebenenfalls einfach bis zweifach, gleich oder verschieden durch Fluor, Chlor, Methyl oder Trifluormethyl substituiertes Benzyl steht.

Bevorzugte erfindungsgemäße Verbindungen sind auch Additionsprodukte aus Säuren und denjenigen Hydroxyethylazoly-oxim-Derivaten der Formel (I), in denen die Substituenten Ar, $R^1$ und $R^2$ die Bedeutungen haben, die bereits vorzugsweise für diese Substituenten gennant wurden.

Zu den Säuren, die addiert werden können, gehören vorzugszweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II, bis IV. Haupt- und der I, und II, sowie IV, bis VIII. Nebengruppe und denjenigen Hydroxyethylazolyl-oxim-Derivaten der Formel (I), in denen die Substituenten Ar, $R^1$ und $R^2$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie die z.B. Chlorwasserstoffsäure und die Bromwasserstoffsäure, Salpetersäure und Schwefelsäure.

Im einzelnen seien außer den bei den Herstellungsbeispielen genannten Verbindungen die folgenden Verbindungen der allgemeinen Formel

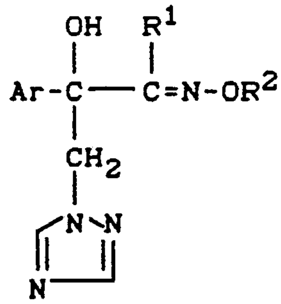

(I)

genannt:

| Ar | $R^1$ | $R^2$ |
|---|---|---|
| 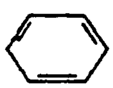 | H | $CH_3$ |
| 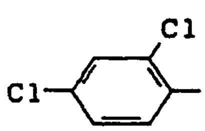 | H | $C_2H_5$ |
| 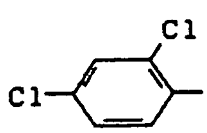 | H | $C_4H_9$-n |
| 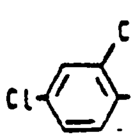 | H | $C_3H_7$ |

| Ar | $R^1$ | $R^2$ |
|---|---|---|
| 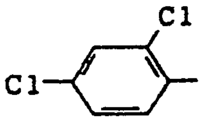 | H | 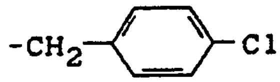 |
| 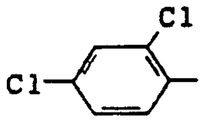 | $C_4H_9$-n | H |
| 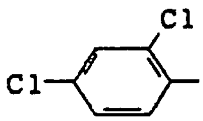 | $C_4H_9$ -n | $CH_3$ |
| 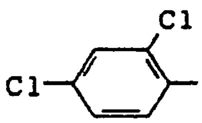 | $C_3H_7$-n | $CH_3$ |
| 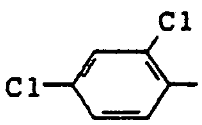 | $CH_3$ | $CH_3$ |
| 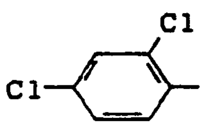 | $CH_3$ | $C_2H_5$ |
| 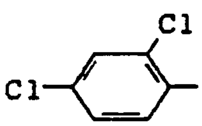 | $CH_3$ | $C_3H_7$ |
| 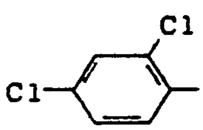 | $CH_3$ | $C_4H_9$-n |
| 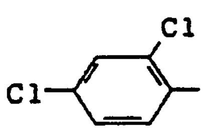 | $CH_3$ | $C_4H_9$-sec. |

| Ar | $R^1$ | $R^2$ |
|---|---|---|
| 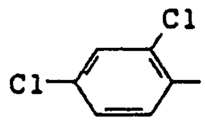 | H | 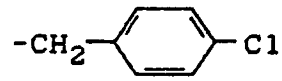 |
| 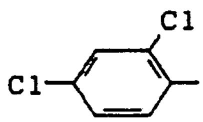 | H | 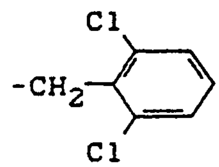 |
| 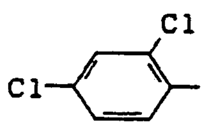 | H | 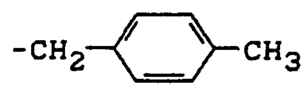 |
| 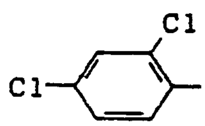 | H | 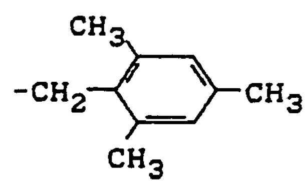 |
| 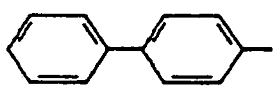 | H | $CH_3$ |
| 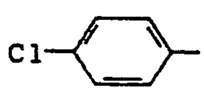 | H | 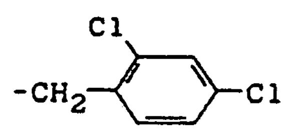 |
| 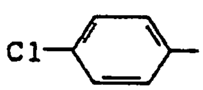 | H | $CH_3$ |

Verwendet man beispielsweise 2-(2,4-Dichlorphenyl)-3,3-dimethoxy-2-hydroxy-1-(1,2,4-triazol-1-yl)-propan und O-Methyl-hydroxylamin-hydrochlorid als Ausgangsstoffe, so kann der Ablauf des erfindungsgemäßen Verfahrens (a) durch das folgende Formelschema wiedergegeben werden:

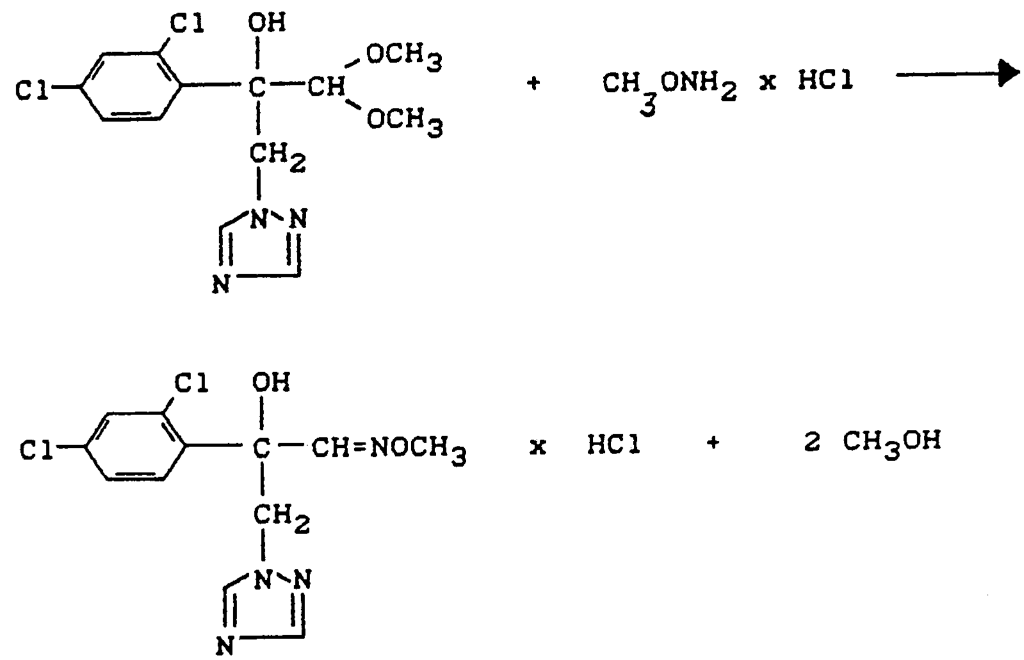

Verwendet man beispielsweise 2-(2,4-Dichlorphenyl)-2-hydroxy-2-hydroxyiminomethyl-1-(1,2,4-triazol-1-yl)-ethan und 4-Chlorbenzylchlorid als Ausgangsstoffe, so kann der Ablauf des erfindungsgemäßen Verfahrens (b) durch das folgende Formelschema wiedergegeben werden:

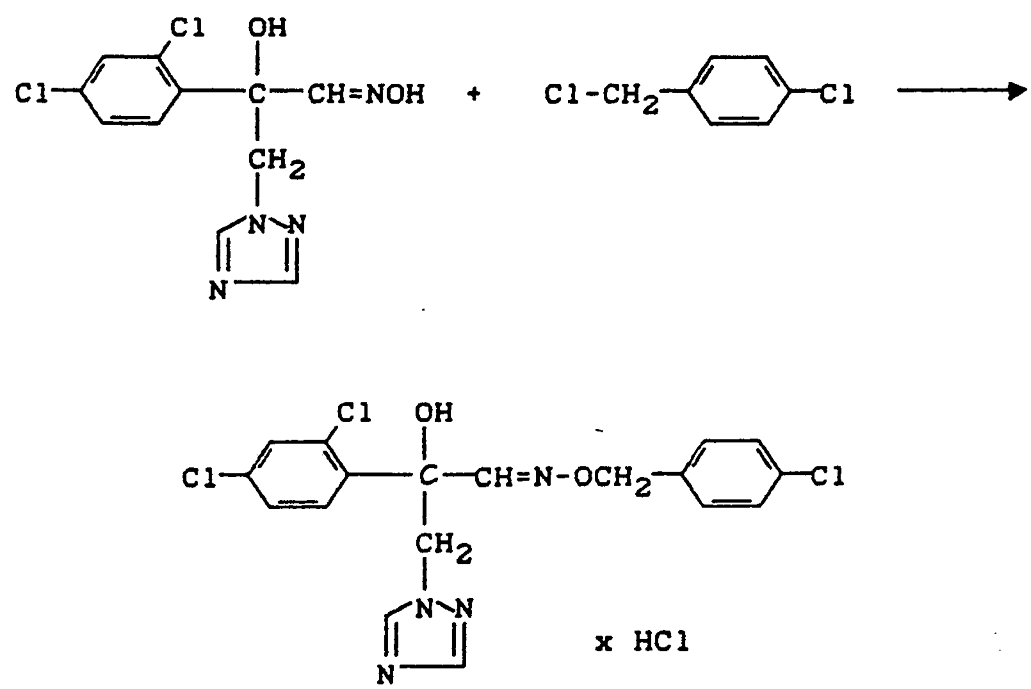

Verwendet man beispielsweise 2-(2,4-Dichlorphenyl)-2-(1-methoxyimino-1-butyl)-oxiran und 1,2,4-Triazol als Ausgangsstoffe, so kann der Ablauf des erfindungsgemäßen Verfahrens (c) durch das folgende Formelschema wiedergegeben werden:

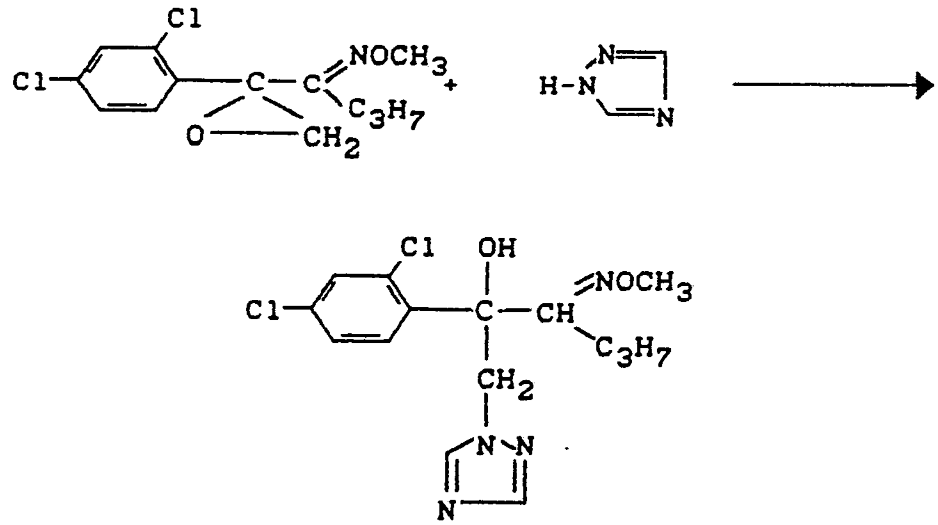

Die für die Durchführung des erfindungsgemäßen Verfahrens (a) als Ausgangsstoffe zu verwendenden Hydroxyethylazolyl-keto-Derivate sind durch die Formel (II) allgemein definiert. In dieser Formel haben Ar, $R^1$ und $R^2$ vorzugsweise die Bedeutungen, die bereits im Zusammenhang mit der Beschreibung der erfindungsgemäßen Stoffe der Formel (I) vorzugsweise für diese Substituenten genannt wurden.

Die Hydroxyethylazolyl-keto-Derivate der Formel (II) bzw. deren Ketale und Acetale sind teilweise beschrieben (vgl. EP—A 0 078 594 und DE—A 3 242 252). Sie lassen sich nach den dort angegebenen Verfahren erhalten, indem man Oxirane der Formel

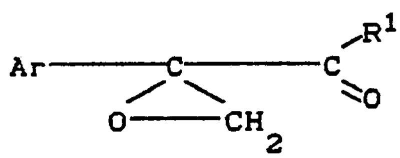

(VII)

in welcher

Ar und $R^1$ die oben angegebene Bedeutung haben, wobei die Keto-Gruppe auch in ketten- oder ringförmiger Acetal- bzw. Ketal-Struktur vorliegen kann,

mit Azolen der Formel (VI) entsprechend den Bedingungen des Verfahrens (c) umsetzt.

Die Oxirane der Formel (VII) sind teilweise beschrieben (vgl. EP—A 0 078 549 bzw. DE—A 3 242 252). Sie können in allgemein bekannter Art und Weise erhalten werden, indem man die entsprechenden Keton ein üblicher Weise epoxidiert.

Die außerdem für die Durchführung des erfindungsgemäßen Verfahrens (a) als Ausgangsstoffe zu verwendenden Hydroxyl-amin-Derivate sind durch die Formel (III) allgemein definiert. In dieser Formel steht $R^2$ vorzugsweise für die Bedeutungen, die bereits im Zusammenhang mit der Beschriebung der erfindungsgemäßen Stoffe der Formel (I) vorzugsweise für diesen Substituenten genannt wurden.

Die Hydroxylamin-Derivate der Formel (III) sind allgemein bekannte Verbindungen der organischen Chemie.

Die bei der Durchführung des erfindungsgemäßen Verfahrens (b) als Ausgangsstoffe zu verwendenden Hydroxyethylazolyl-oxim-Derivate der Formel (Ia) sind erfindungsgemäße Verbindungen.

Die außerdem für die Durchführung des erfindungsgemäßen Verfahrens (b) als Ausgangsstoffe zu verwendenden Halogenide sind durch die Formel (IV) allgemein definiert. In dieser Formel steht $R^3$ vorzugsweise für die Bedeutungen, die bereits im Zusammenhang mit der Beschreibung der erfindungsgemäßen Stoffe der Formel (I) für $R^2$, außer Wasserstoff, vorzugsweise genannt wurden.

Die Halogenide der Formel (IV) sind allgemein bekannte Verbindungen der organischen Chemie.

Die bei der Durchführung des erfindungsgemäßen Verfahrens (c) als Ausgangsstoffe zu verwendenden Oxirane sind durch die Formel (V) allgemein definiert. In dieser Formel stehen Ar, $R^1$ und $R^3$ vorzugsweise für die Bedeutungen, die bereits im Zusammenhang mit der Beschreibung der erfindungsgemäßen Stoffe der Formel (I) bzw. der Halogenide der Formel (IV) vorzugsweise für diese Substituenten genannt wurden.

Die Oxirane der Formel (V) sind noch nicht bekannt. Sie können jedoch in allgemein bekannter Art und Weise erhalten werden, indem man die entsprechenden Keto-oxim-Derivate der Formel

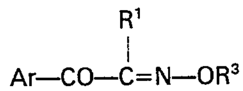

(VIII)

in welcher

Ar, $R^1$ und $R^2$ die oben angegebene Bedetung haben, entweder

α) mit Dimethyloxosulfonium-methylid der Formel

$$\overset{\delta^+}{(CH_3)_2SO}\overset{\delta^-}{CH_2} \quad (IX)$$

in an sich bekannter Weise in Gegenwart eines Verdünnungsmittels, wie beispielsweise Dimethylsulfoxid, bei Temperaturen zwischen 20°C und 80°C umsetzt (vgl. hierzu die Angaben in J. Am. Chem. Soc. *87*, 1363—1364 (1965)) oder

β) mit Trimethylsulfonium-methylsulfat der Formel

$$[(CH_3)_3S^{\oplus}]CH_3SO_4^{\ominus} \quad (X)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0°C bis 60°C, vorzugsweise bei Raumtemperatur, umsetzt (vgl. auch die Angaben in Heterocycles *8*, 397 (1977)).

Die Keto-oxim-Derivate der Formel (VIII) werden erhalten, indem man entsprechende Ketone der Formel

$$Ar{-}CO{-}CH_2{-}R^1 \quad (XI)$$

in welcher

Ar und $R^1$ die oben angegebene Bedeutung haben, in üblicher Art und Weise mit einem nitrosierenden Agenz, wie beispielsweise salpetriger Säure und ihrer Ester, umsetzt und gegebenenfalls die entstehenden Ketooxime der Formel

$$Ar{-}CO{-}\underset{}{\overset{R^1}{\overset{|}{C}}}{=}N{-}OH \quad (XII)$$

in welcher

Ar und $R^1$ die oben angegebene Bedeutung haben,

mit Halogeniden der Formel (IV) entsprechend den Bedingungen des Verfahrens (b) umsetzt.

Die außerdem für die Durchführung des erfindungsgemäßen Verfahrens (c) als Ausgangsstoffe zu verwendenden Azole sind durch die Formel (VI) allgemein definiert. In dieser Formel steht M vorzugsweise für Wasserstoff, Natrium oder Kalium.

Die Azole der Formel (VI) sind allgemein bekannte Verbindungen der organischen Chemie.

Als Verdünnungsmittel kommen für das erfindungsgemäße Verfahren (a) vorzugsweise Alkohole und Wasser, bzw. Gemische beider infrage.

Die Reaktionstemperaturen können beim Verfahren (a) in einem größeren Bereich variiert werden. Im allgemeinen arbeitet man zwischen 20°C und 120°C, vorzugsweise zwischen 50°C und 100°C.

Bei der Durchführung des erfindungsgemäßen Verfahrens (a) setzt man auf 1 Mol der Verbindung der Formel (II) vorzugsweise 1 bis 1,5 Mol Hydroxylamin-Derivat der Formel (III) ein. Die Isolierung der Verbindungen der Formel (I) erfolgt nach üblichen Methoden.

Nach einer bevorzugten Ausführungsform des Verfahrens (a) werden die Hydroxylamin-Derivate der Formel (III) in Form ihrer Salze, insbesondere als Hydrochloride, gegebenenfalls in Gegenwart eines Säurebindemittels, wie beispielsweise Natriumacetat, eingesetzt.

Für die erfindungsgemäße Umsetzung gemäß Verfahren (b) kommen als Verdünnungsmittel inerte organischen Lösungsmittel infrage. Hierzu gehören vorzugsweise Ether, wie Tetrahydrofuran und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Hexamethylphosphorsäure-triamid, Säureamide, wie Dimethylformamid und Sulfoxide, wie Dimethylsulfoxid.

Die erfindungsgemäße Umsetzung gemäße Verfahren (b) wird gegenbenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyltrimethylammoniumhydroxid oder Dibenzyldimethyl-ammoniumhydroxid und Tetraphenylphosphoniumhydroxid oder Methyltriphenylphosphoniumhydroxid.

Die Reaktionstemperaturen können bei dem Verfahren (b) in einem größeren Bereich variiert werden. Im allgemeinen arbeitet man zwischen 20°C und 150°C, vorzugsweise bei Raumtemperatur. In einzelnen Fällen ist es vorteilhaft, bei der Siedetemperatur des Lösungsmittels, beispielsweise zwischen 60°C und 100°C, zu arbeiten.

Bei der Durchführung des erfindungsgemäßen Verfahren (b) setzt man auf 1 Mol der Verbindungen der

Formel (Ia) vorzugsweise 1 bis 3 Mol Halogenid der Formel (IV) ein. Zur Isolierung der Endprodukte wird das Reaktionsgemisch vom Lösungsmittel befreit und der Rückstand mit Wasser und einem organischen Lösungsmittels versetzt. Die organische Phase wird abgetrennt, in üblicher Weise aufgearbeitet und gereinigt.

In einer bevorzugten Ausführungsform des Verfahrens (b) wird die erfindungsgemäße Umsetzung in eines Zweiphasensystem, wie beispielsweise wässrige Natron- oder Kalilauge/Toluol oder Methylenchlorid, unter Zusatz von 0,01 bis 1 Mol eines Phasen-Transfer-Katalysators, wie beispielsweise Ammonium- oder Phosphoniumverbindungen, durchgeführt, wobei in der organischen Phase oder an der Grenzfläche die Alkoholate entstehen und mit den in der organischen Phase befindlichen Halogeniden umgesetzt werden.

Als Verdünnungsmittel kommen für das erfindungsgemäße Verfahren (c) unter den Reaktionsbedingungen inerte organische Lösungsmittel infrage. Hierzu gehören vorzugsweise Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserestoffe, wie. z.B. Benzol und Toluol; oder Amide, wie z.B. Dimethylformamid.

Als Basen kommen für die erfindungsgemäße Umsetzung gemäß Verfahren (c) alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie. z.B. Natriumhydroxid; Alkalialkoholate, wie z.B. Natrium- und Kalium-methylat und -ethylat; Alkalihydride, wie. z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens (c) in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens (c) setzt man vorzugsweise auf 1 Mol Oxiran der Formel (V) 1 bis 2 Mol Azol der Formel (V) und gegebenenfalls 1 bis 2 Mol Base ein; die Isolierung der Endprodukte erfolgt in allgemeinen üblicher Weise.

Zur Herstellung von Säureadditions-Salzen der Verbindungen der allgemeinen Formel (I) kommen vorzugsweise diejenigen Säuren infrage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organichen Lösungsmittel gereinigt werden.

Zur Herstellung von Metallsalz-Komplexen der Verbindungen der allgemeinen Formel (I) kommen vorzugsweise diejenigen Salze von Metallen infrage, die bereits weiter oben beschrieben wurden.

Die Metallsalz-Komplexe von Verbindungen der allgemeinen Formel (1) können in einfacher Weise nach üblichen Verfahren erhalten werden, so z.B. durch Lösen des Metallsalzes in Alkohol, z.B. Ethanol und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel in Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die Verbindungen zeigen eine gute Wirkung insbesondere gegen Puccinia-Arten und Cochliobolus-Arten in Weizenkulturen, sowie gegen Venturia-Arten, wie z.B. gegen Venturia inaequalis, den Erreger des Apfelschorfes. Außerdem ist noch zu erwähnen eine gute Wirkung gegen Mehltau und Septoria nodorum an Getreide und gegen Pyricularia oryzae an Reis. Im Agarplattentest zeigen die erfindungsgemäßen Wirkstoffe eine größe Wirkungsbreite.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsions-konzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfs lösungsmittel verwendet

werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive konnen mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschiet in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einen größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Verwendungsbeispiele

In den nachfolgenden Beispielen werden die nachstehend angegebenen Verbindungen als Vergleichssubstanzen eingesetzt:

(A)

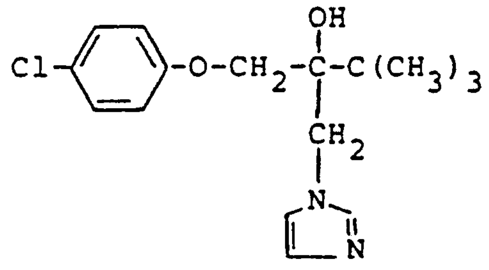

(B)

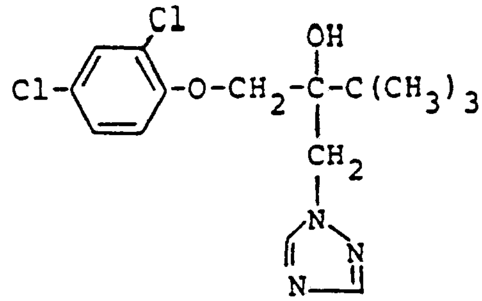

(C)

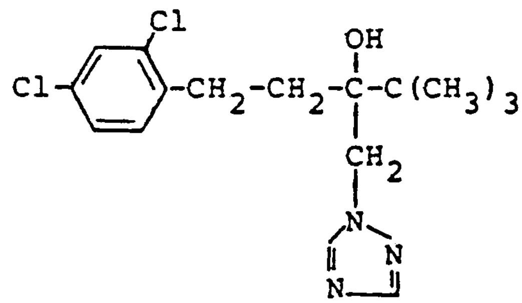

(D)

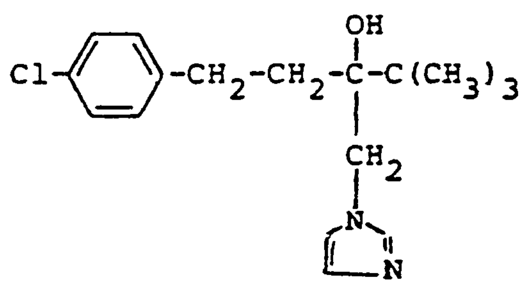

Beispiel A

Puccinia-Test (Weizen)/protektiv

Lösungsmittel: 100 Gewichtsteile Dimethylformamid

Emulgator: 0,25 Gewichtsteile Alhylarylpolyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen. 10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2, 4 und 6.

Beispiel B

Cochliobolus sativus-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrockenen des Spritzbelages werden die Pflanzen mit einer Konidiensuspension von

Cochliobolus sativus besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele: 2, 4 und 6.

Beispiel C

Venturia-Test (Apfel)/protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichsteil Wirkstoff mit den angegebenen Mengen Lösungsmittel und Emulgator und verdünnt des Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer Konidiensuspension des Apfelschorferregers (Venturia inaequalis) inokuliert und verbleiben dann 1 Tage bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 2 und 4.

Herstellungsbeispiele:

Beispiel 1

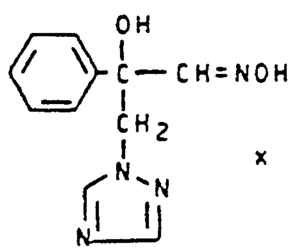

x HCl

(Verfahren (a))

10 g (0,038 Mol) 1,1-Dimethoxy-2-hydroxy-2-phenyl-3-(1,2,4-triazol-1-yl)-propan werden mit 50 ml Wasser, 5 ml konzentrierter Salzsäure und 3 g Hydroxylammonium-hydrochlorid 15 Stunden unter Rückfluß erhitzt. Anschließend wird das Reaktionsgemisch mit gesättigter Natriumhydrogencarbonatlösung versetzt. Die wässrige Phase wird mit 100 ml n-Butanol versetzt, die organische Phase wird danach abgetrennt und die wässrige Phase zweimal mit je 50 ml Butanol gewaschen. Die vereinigten Butanolphasen werden mit 50 ml Wasser gewaschen, über Natriumsulfat getrocknet und im Vakuum eingeengt. Der Rückstand wird in 100 ml Methylenchlorid aufgenommen und mit 50 ml mit Chlorwasserstoff gesättigtem Diethylether versetzt. Das Reaktionsgemisch wird eingeengt und der Rückstand aus 100 ml Acetonitril umkristallisiert.

Man erhält 4 g (40% der Theorie) 2-Hydroxy-2-phenyl-2-(1,2,4-triazol-1-yl-methyl)-acetaldoxim-hydrochlorid vom Schmelzpunkt 178°C bis 180°C (Zersetzung).

Herstellung des Ausgangsproduktes:

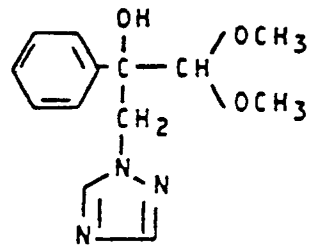

Durch die übliche Umsetzung von rohem 2-Dimethoxymethyl-2-phenyl-oxiran mit 1,2,4-Triazol in Gegenwart von Kaliumhydroxid erhält man 1,1-Dimethoxy-2-hydroxy-2-phenyl-3-(1,2,4-triazol-1-yl)-propan von Schmelzpunkt 85°C bis 86°C.

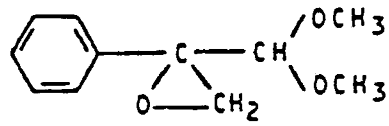

Durch übliche Umsetzung von ω-Dimethoxyacetophenon mit Dimethylsulfid/Dimethylsulfat in Gegenwart von Kalium-tert.-butylat erhält man 2-Dimethoxymethyl-2-phenyl-oxiran, das direkt weiter umgesetzt wird.

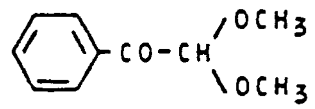

Durch übliche Umsetzung von Dimethoxy-phenyl-acetaldehyd in Methanol mit konzentrierter Salzsäure erhält man ω-Dimethoxyacetophenon vom Siedepunkt 130°C/8 Torr.

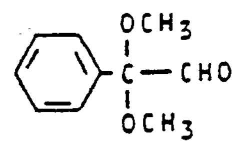

Durch übliche Umsetzung von ω-Dichloracetophenon mit Natriummethylat in Methanol erhält man Dimethoxy-phenyl-acetaldehyd, der direkt weiter umgestezt wird.

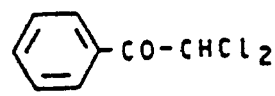

Durch übliche Umsetzung von Acetophenon mit Sulfurylchlorid erhält man ω-Dichloracetophenon vom Siedepunkt 130°C/12 Torr.

Beispiel 2

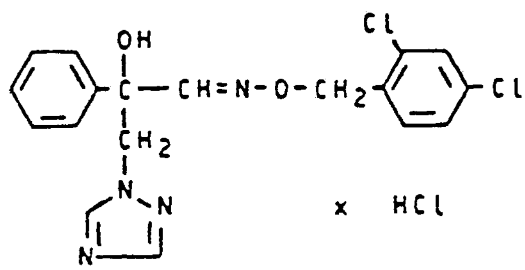

(Verfahren (b))

4 g (0,015 Mol) 2-Hydroxy-2-phenyl-2-(1,2,4-triazol-1-yl-methyl)-acetaldoxim-hydrochlorid (Beispiel 1) in 40 ml Toluol werden mit 40 ml wässriger Natriumhydroxidlösung, 1 ml einer 50% igen wässerigen Lösung von Trimethylbenzyl-ammoniumhydroxid und 2,9 g (0,015 Mol) 2,4-Dichlorbenzylchlorid 10 Stunden bei Raumtemperatur gerührt. Danach wird die Toluolphase abgetrennt, dreimal mit je 100 ml gesättigter Natriumchloridlösung gewaschen, über Natriumsulfat getrocknet und im Vakuum eingeengt. Der Rückstand wird in Diethylether aufgenommen und mit Chlorwasserstoff gesättigtem Diethylether versetzt. Das Reaktionsgemisch wird eingeengt und der Rückstand aus 20 ml Ethanol umkristallisiert.

Man erhält 1 g (15,6% der Theorie) 3-(2,4-Dichlorbenzyloximino)-2-hydroxy-2-phenyl-1-(1,2,4-triazol-1-yl)-propan-hydrochlorid vom Schmelzpunkt 148°C bis 153°C.

In entsprechender Weise und gemäß den angegebenen Verfahren können die folgenden Verbindungen der allgemeinen Formel (I) erhalten werden:

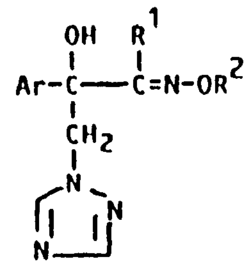

(I)

| Beisp. Nr. | Ar | $R^1$ | $R^2$ | Physikalische Konstante |
|---|---|---|---|---|
| 3 | 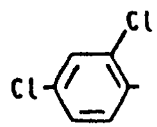 | H | H | Oel (x HCl), $^1HNMR_{CDCl_3/DMSO}$:<br>$\delta$ = 5,23 ppm (s), fOr -C $H_2$ -N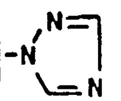<br>$\delta$ = 7,43 ppm (s), für -C H =N- |
| 4 | 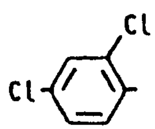 | H | 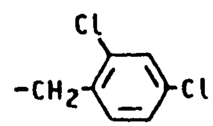 | Fp. = 125-30°C |
| 5 | 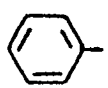 | H | $CH_3$ | Oel (x HCl), $^1HNMR_{DMSO}$:<br>$\delta$ = 4,47 ppm (s), für -C $H_2$ -N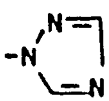<br>$\delta$ = 5,70 ppm (s), für -C H =N- |
| 6 | 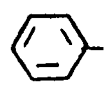 | $CH_3$ | 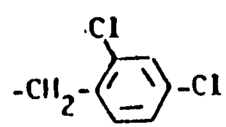<br>x 1/2 NDS | Fp. = 212 - 216°C |

NDS = 1,5-Naphthalindisulfonsäure

| Beisp. Nr | Ar | $R^1$ | $R^2$ | Physikalische Konstante |
|---|---|---|---|---|
| 7 | 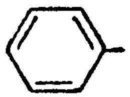 | $CH_3$ | 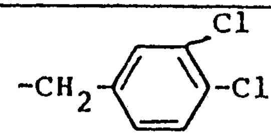 | Fp. 158-160°C (x HCl) |
| 8 | 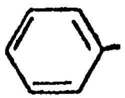 | $CH_3$ | 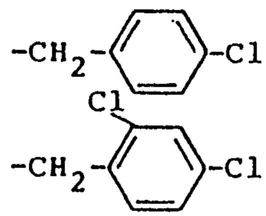 | Fp. 151-157°C (x HCl) |
| 9 | 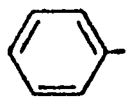 | $CH_3$ | | Öl, $^1HNMR_{CDCl_3}$ : $\delta$ ($CH_3$) = 1,73/1,76 ppm (Isomerengemisch im Verhältnis 1 : 3) |
| 10 | 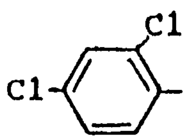 | H | 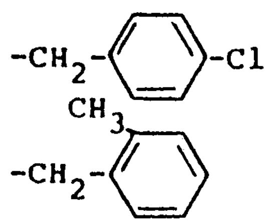 | Fp. 145°C |
| 11 | 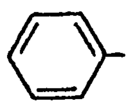 | $CH_3$ | | Fp. 168-178°C (u. Zers.) |
| 12 | 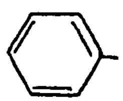 | $CH_3$ | 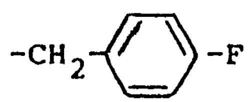 | Fp. 148 - 150°C (xHCl) |

**Patentansprüche**

1. Hydroxyethylazolyl-oxim-Derivate der Formel

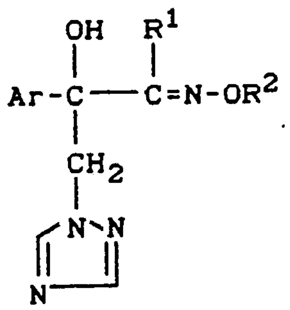

(I)

in welcher

Ar für Phenyl steht, das einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, Alkoximinomethyl mit 1 bis 4 Kohlenstoffatomen im Alkoxyteil, Phenyl und Phenoxy,

$R^1$ für Wasserstoff oder geradkettiges oder verzweigtes Alkyl mit 1 bis 4 Kohlenstoffatomen steht und

$R^2$ für Wasserstoff, geradkettiges oder verzweigtes Alkyl mit 1 bis 4 Kohlenstoffatomen steht oder für Phenylalkyl mit 1 oder 2 Kohlenstoffatomen im Alkylteil steht, wobei der Phenylteil einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, Alkoximinomethyl mit 1 bis 4 Kohlenstoffatomen im Alkoxyteil, Phenyl und Phenoxy,

und deren Säureadditions-Salze und Metallsalz-Komplexe.

2. Verbindungen der Formel (I) in Anspruch 1, wobei

Ar für gegenbenenfalls einfach bis dreifach, gleich oder verschieden durch Fluor, Chlor, Methyl, Trifluormethyl, Methoximinomethyl, Ethoximinomethyl, Phenyl und/oder Phenoxy substituiertes Phenyl steht,

$R^1$ für Wasserstoff oder geradkettiges Alkyl mit 1 bis 4 Kohlenstoffatomen steht und

$R^2$ für Wasserstoff, geradkettiges Alkyl mit 1 bis 4 Kohlenstoffatomen oder für gegebenenfalls einfach bis zweifach, gleich oder verschieden durch Fluor, Chlor, Methyl oder Trifluormethyl substituiertes Benzyl steht.

3. Verfahren zur Herstellung von Hydroxyethylazolyl-oxim-Derivaten der Formel

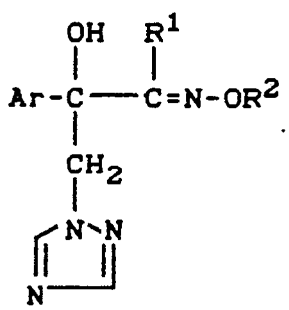

(I)

in welcher

Ar für Phenyl steht, das einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen un 1 bis 5 gleichen oder verschiedenen Halogenatomen, Alkoximinomethyl mit 1 bis 4 Kohlenstoffatomen im Alkoxyteil, Phenyl und Phenoxy,

$R^1$ für Wasserstoff der geradkettiges oder verzweigtes Alkyl mit 1 bis 4 Kohlenstoffatomen steht und

$R^2$ für Wasserstoff, geradkettiges oder verzweigtes Alkyl mit 1 bis 4 Kohlenstoffatomen steht oder für Phenylalkyl mit 1 oder 2 Kohlenstoffatomen im Alkylteil steht, wobei der Phenylteil einfach oder mehrfach, gleich oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, Alkoximinomethyl mit 1 bis 4 Kohlenstoffatomen im Alkoxyteil, Phenyl und Phenoxy,

und von deren Säureadditions-Salzen und Metallsalz-Komplexen,

dadurch gekennzeichnet, daß man

a) Hydroxyethylazolyl-keto-Derivate der Formel

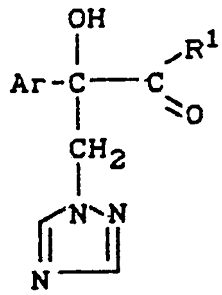

(II)

in welcher

Ar und $R^1$ die oben angegebene Bedeutung haben, wobei die Keto-Gruppe auch in Ketten- oder ringförmiger Acetal- bzw. Ketal-Struktur vorliegen kann,

mit Hydroxylamin-Derivaten der Formel

$$H_2N—O—R^2 \qquad (III)$$

in welcher

$R^2$ die oben angegebene Bedeutung hat,

in Gegenwart eines Verdünnungsmittels umsetzt, und gegebenenfalls noch

b) nach Verfahren a) erhaltene Hydroxyethyl-azolyloxim-Derivate der Formel

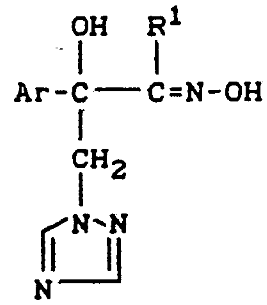

(Ia)

in welcher

Ar und $R^1$ die oben angegebene Bedeutung haben, mit Halogeniden der Formel

$$Hal—R^3 \qquad (IV)$$

in welcher

Hal für Chlor, Brom oder Jod steht und

$R^3$ für die Bedeutungen von $R^2$, außer für Wasserstoff, steht,

in Gegenwart eines Verdünnungsmittels und gegebenenfalls in Gegenwart einer Base umsetzt; oder

c) Oxirane der Formel

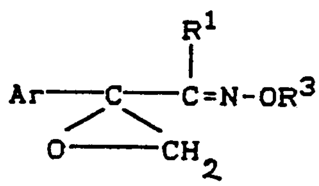

(V)

in welcher

Ar, $R^1$ und $R^3$ die oben angegebene Bedeutung haben,

mit Azolen der Formel

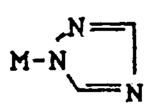

(VI)

in welcher

M für Wasserstoff oder ein Alkalimetall steht,

in Gegenwart eines Verdünnungsmittels und gegebenenfalls in Gegenwart einer Base umsetzt, und gegebenenfalls noch an die so erhaltenen Verbindungen der Formel (I) anschließend eine Säure oder ein Metallsalz addiert.

4. Fungizide Mittel, gekennzeichnet durch einen Gehalt an mindestens ein Hydroxyethylazolyl-oxim-Derivat der Formel (I) in Anspruch 1 bzw. an einem Säureadditions-Salz oder Metallsalz-Komplex eines Hydroxyethylazolyl-oxim-Derivates der Formel (I).

5. Verfahren zur Bekämpfung von Pilzen, dadurch gekennzeichnet, daß man Hydroxyethylazolyl-oxim-Derivat der Formel (I) in Anspruch 1 bzw. deren Säureadditions-Salze oder Metallsalz-Komplexe auf Pilze oder ihren Lebenraum einwirken läßt.

6. Verwendung von Hydroxyethylazolyl-oxim-Derivaten der Formel (I) in Anspruch 1 bzw. deren Säureadditions-Salzen oder Metallsalz-Komplexen als Pflanzenschutzmittel.

7. Verwendung von Hydroxyethylazolyl-oxim-Derivaten der Formel (I) in Anspruch 1 bzw. deren Säureadditions-Salzen oder Mettalsalz-Komplexen zur Bekämpfung von Pilzen.

8. Verfahren zur Herstellung von fungiziden Mitteln, dadurch gekennzeichnet, daß man Hydroxyethylazolyl-oxim-Derivate der Formel (I) in Anspruch 1 bzw. deren Säureadditions-Salze oder Metallsalz-Komplexe mit Streckmitteln und/oder oberflächenaktiven Mitteln vermischt.

**Revendications**

1. Dérivés d'hydroxyéthlyazolyloximes de formule

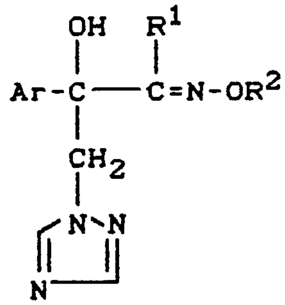

(I)

dans laquelle

Ar est un groupe phényle qui peut porter un ou plusieurs substituants, identiques ou différents, halogéno, alkyle ayant 1 à 4 atomes de carbone, alkoxy ayant 1 ou 2 atomes de carbone, alkylthio ayant 1 ou 2 atomes de carbone halogénalkyle ayant 1 ou 2 atomes de carbone et 1 à 5 atomes d'halogènes identiques ou différents, alkoximinoéthyle ayant 1 à 4 atomes de carbone dans la partie alkoxy, phényle et phénoxy,

$R^1$ est l'hydrogène ou un groupe alkyle à chaîne droite ou à chaîne ramifiée ayant 1 à 4 atomes de carbone, et

$R^2$ est l'hydrogène, un groupe alkyle à chaîne droite ou à chaîne ramifiée ayant 1 à 4 atomes de carbone ou un groupe phénylalkyle ayant 1 ou 2 atomes de carbone dans la partie alkyle, le partie phényle pouvant porter un ou plusieurs substituants, identiques ou différents, halogéno, alkyle ayant 1 à 4 atomes de carbone, alkoxy ayant 1 ou 2 atomes de carbone, alkylthio ayant 1 ou 2 atomes de carbone, halogénalkyle ayant 1 ou 2 atomes de carbone et 1 à 5 atomes d'halogènes identiques ou différents, alkoximinométhyle ayant 1 à 4 atomes de carbone dans la partie alkoxy, phényle et phénoxy.

et leurs sels d'addition d'acides et leurs complexes de sels métalliques.

2. Composés de formule (I) suivant la revendication 1, dans lesquels

Ar désigne un groupe phényle portant éventuellement 1 à 3 substituants, identiques ou différents, fluoro, chloro, méthyle, trifluorométhyle, méthoximinométhyle, éthoximinométhyle, phényle et/ou phénoxy,

$R^1$ est l'hydrogène ou un groupe alkyle à chaîne droite ayant 1 à 4 atomes de carobone, et

$R^2$ désigne l'hydrogène, un groupe alkyle à chaîne droite ayant 1 à 4 atomes de carbone ou un groupe benzyle portant éventuellement 1 ou 2 substituants fluoro, chloro, méthyle ou trifluorométhyle identiques ou différents.

3. Procédé de production de dérivés d'hydroxyéthylazolyloximes de formule

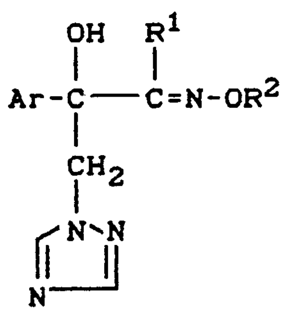

(I)

dans laquelle

Ar est un groupe phényle qui peut porter un ou plusieurs substituants, identiques ou différents, halogéno, alkyle ayant 1 à 4 atomes de carbone, alkoxy ayant 1 ou 2 atomes de carbone, alkylthio ayant 1

ou 2 atomes de carbone, halogénalkyle ayant 1 ou 2 atomes de carbone et 1 à 5 atomes d'halogènes identiques ou différents, alkoximinométhyle ayant 1 à 4 atomes de carbone dans la partie alkoxy, phényle et phénoxy,

$R^1$ est l'hydrogéne ou un groupe alkyle à chaîne droite ou à chaîne ramifiée ayant 1 à 4 atomes de carbone et

$R^2$ est l'hydrogène, un groupe alkyle à chaîne droite ou à chaîne ramifiée ayant 1 à 4 atomes de carbone ou un groupe phénylalkyle ayant 1 ou 2 atomes de carbone dans la partie alkyle, le partie phényle pouvant porter un ou plusieurs substituants, identiques ou différents, halogéno, alkyle ayant 1 à 4 atomes de carbone, alkoxy ayant 1 ou 2 atomes de carbone, alkylthio ayant 1 ou 2 atomes de carbone, halogénalkyle ayant 1 ou 2 atomes de carbone et 1 à 5 atomes d'halogènes identiques ou différents, alkoximinométhyle ayant 1 à 4 atomes de carbone dans la partie alkoxy, phényle et phénoxy,

et de leurs sels d'addition d'acides et de leurs complexes de sels métalliques,

caractérisé en ce que

a) on fait réagir des dérivés hydroxyéthylazolyl-cétoniques de formule

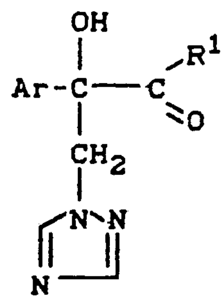

(II)

dans laquelle

Ar et $R^1$ ont la définition indiquée ci-dessus, le groupe céto pouvant aussi être présent dans la structure d'acétal ou de cétal en chaîne ou cyclique,

avec des dérivés d'hydroxylamine de formule

$$H_2N—O—R^2 \quad (III)$$

dans laquelle $R^2$ a la définition indiquée ci-dessus,

en présence d'un diluant, et le cas échéant

b) on fait réagir des dérivés d'hydroxyéthylazolyloximes obtenus selon le procédé a) de formule

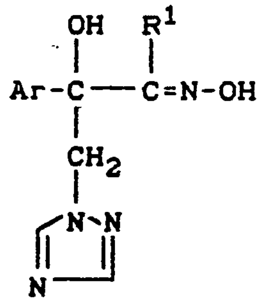

(Ia)

dans laquelle

Ar et $R^1$ ont la définition indiquée ci-dessus, avec des halogénures de formule

$$Hal—R^3 \quad (IV)$$

dans laquelle Hal désigne le chlore, le brome ou l'iode, et

$R^3$ a les définitions de $R^2$, hormis l'hydrogène,

en présence d'un diluant et, le cas échéant, en présence d'une base; ou bien

c) on fait réagir des oxirannes de formule

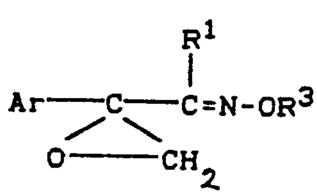

(V)

dans laquelle

Ar, $R^1$ et $R^3$ ont la définition indiquée ci-dessus,

avec des azoles de formule

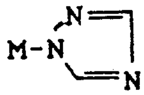

(VI)

dans laquelle

M désigne l'hydrogène ou un métal alcalin,

en présence d'un diluant et, le cas échéant, en présence d'une base, et on additionne ensuite éventuellement un acide ou un sel métallique sur les composés de formule (I) anisi obtenus.

4. Compositions fongicides, caractérisées par une teneur en au moins un dérivé d'hydroxyéthyl-azolyloxime de formule (I) suivant la revendication 1 ou en un sel d'addition d'acide ou un complexe de sel métallique d'une dérivé d'hydroxyéthylazolyloxime de formule (I).

5. Procédé pour combattre des champignons, caractérisé en ce qu'on fait agir des dérivés d'hydroxy-éthylazolyloximes de formule (I) suivant la revendication 1 ou leurs sels d'addition d'acides ou, de leurs complexes de sels métalliques sur des champignons ou sur leur milieu.

6. Utilisation de dérivés d'hydroxyéthylazolyloxime de formule (I) suivant la revendication 1 ou de leurs sels d'addition d'acides ou de leurs complexes de sels métalliques comme agents pour la protection des plantes.

7. Utilisation de dérivés d'hydroxyéthylazolyloxime de formule (I) suivant la revendication 1 ou de leurs sels d'addition d'acides ou de leurs complexes de sels métalliques pour combattre des champignons.

8. Procédé de préparation de compositions fongicides, caractérisé en ce qu'on mélange des dérivés d'hydroxyéthylazolyloximes de formule (I) suivant la revendication 1 ou leurs sels d'addition d'acides ou leurs complexes de sels métalliques avec des diluants et/ou des agents tensio-actifs.

**Claims**

1. Hydroxyethylazolyl-oxime derivatives of the formula

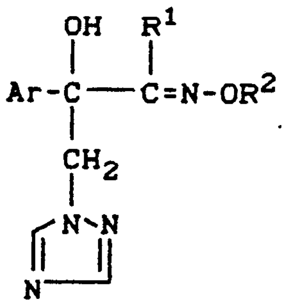

(I)

in which

Ar represents phenyl which can be monosubstituted or polysubstituted by identical or different substituents, substituents which may be mentioned being: halogen, alkyl with 1 to 4 carbon atoms, alkoxy with 1 or 2 carbon atoms, alkylthio with 1 or 2 carbon atoms, halogenoalkyl with 1 or 2 carbon atoms and 1 to 5 identical or different halogen atoms, alkoximinomethyl with 1 to 4 carbon atoms in the alkyl part, phenyl and phenoxy,

$R^1$ represents hydrogen or straight-chain or branched alkyl with 1 to 4 carbon atoms and

$R^2$ represents hydrogen, straight-chain or branched alkyl with 1 to 4 carbon atoms, ore represents phenylalkyl which has 1 or 2 carbon atoms in the alkyl part and can be monosubstituted or polysubstituted in the phenyl part by identical or different substituents, substituents which may be mentioned being halogen, alkyl with 1 to 4 carbon atoms, alkoxy with 1 or 2 carbon atoms, alkylthio with 1 or 2 carbon atoms, halogenoalkyl with 1 or 2 carbon atoms and 1 to 5 identical or different halogen atoms, alkoximinomethyl with 1 to 4 carbon atoms in the alkoxy part, phenyl and phenoxy,

and acid addition salts and metal salts complexes thereof.

2. Compounds of the formula (I) in Claim 1, where

Ar represents phenyl which is optionally mono-, di- or trisubstituted by identical or different substituents from the group comprising fluorine, chlorine, methyl, trifluoromethyl, mexthoximinomethy, ethoximinomethyl, phenyl and/or phenoxy,

$R^1$ represents hydrogen or straight-chain alkyl with 1 to 4 carbon atoms and

$R^2$ represents hydrogen, straight-chain alkyl with 1 to 4 carbon atoms, or represents benzyl which is optionally mono- or di-substituted by identical or different substituents from the group comprising fluorine, chlorine, methyl or trifluoromethyl.

3. Process for the preparation of hydroxyethylazolyl-oxime derivatives of the formula

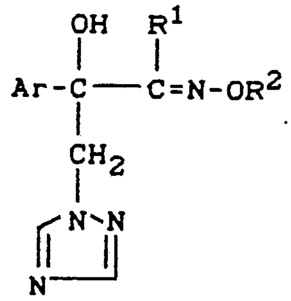

(I)

in which

Ar represents phenyl which can be monosubstituted or polysubstituted by identical or different substituents, substituents which may be mentioned being: halogen, alkyl with 1 to 4 carbon atoms, alkoxy with 1 or 2 carbon atoms, alkylthio with 1 or 2 carbon atoms, halogenoalkyl with 1 or 2 carbon atoms and 1 to 5 identical or different halogen atoms, alkoximinomethyl with 1 to 4 carbon atoms in the alkoxy part, phenyl and phenoxy,

$R^1$ represents hydrogen or straight-chain or branched alkyl with 1 to 4 carbon atoms and

$R^2$ represents hydrogen, straight-chain or branched alkyl with 1 to 4 carbon atoms, or represents phenylalkyl which has 1 or 2 carbon atoms in the alkyl part and can be monosubstituted or polysubstituted in the phenyl part by identical or different substituents, substituents which may be mentioned being halogen, alkyl with 1 to 4 carbon atoms, alkoxy with 1 or 2 carbon atoms, alkylthio with 1 or 2 carbon atoms, halogenoalkyl with 1 or 2 carbon atoms and 1 to 5 identical or different halogen atoms, alkoximinomethyl with 1 to 4 carbon atoms in the alkoxy part, phenyl and phenoxy,

and of acid addition salts and metal salt complexes thereof,

characterized in that

a) hydroxyethylazolyl-keto derivatives of the formula

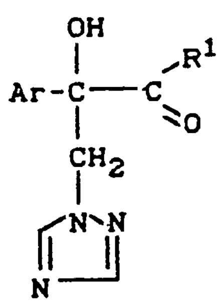

(II)

in which

Ar and $R^1$ have the abovementioned meaning, it also being possible for the keto group to be in a chain-like or cyclic acetal or ketal structure,

and reacted with hydroxylamine derivatives of the formula

$$H_2N—O—R^2 \quad (III)$$

in which

$R^2$ has the abovementioned meaning, in the presence of a diluent, and if appropriate, furthermore,

b) hydroxyethylazolyl-oxime derivatives, obtained according to process a), of the formula

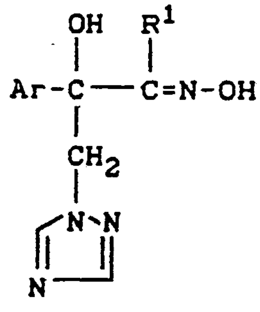

(Ia)

in which

Ar and $R^1$ have the abovementioned meaning, are reacted with halides of the formula

$$Hal—R^3 \qquad (IV)$$

in which

Hal represents chlorine, bromine or iodine and

$R^3$ has the meaning of $R^2$, with the exception of hydrogen,

in the presence of a diluent and if appropriate in the presence of a base; or

c) oxiranes of the formula

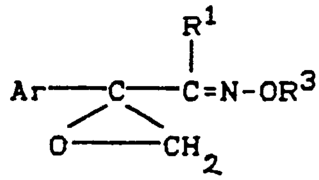

(V)

in which

Ar, $R^1$ and $R^3$ have the abovementioned meaning, are reacted with azoles of the formula

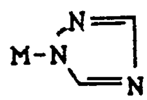

(VI)

in which

M represents hydrogen or an alkali metal, in the presence of a diluent and if appropriate in the presence of a base, and, if appropriate, an acid or a metal salt is then also added onto the compounds of the formula (I) thus obtained.

4. Fungicidal agents, characterized in that they contain at least one hydroxyethylazolyl-oxime derivative of the formula (I) in claim 1 or an acid addition salt or metal salt complex of a hydroxyethylazolyl-oxime derivative of the formula (I).

5. Method of combating fungi, characterized in that hydroxyethylazolyl-oxime derivatives of the formula (I) in Claim 1 or their acid addition salts or metal salt complexes are allowed to act on fungi or their environment.

6. Use of hydroxyethylazolyl-oxime derivatives of the formula (I) in Claim 1 or their acid addition salts or metal salt complexes as plant protection agents.

7. Use of hydroxyethylazolyl-oxime derivatives of the formula (I) in Claim 1 or their acid addition salts or metal salt complexes for combating fungi.

8. Proces for the preparation of fungicidal agents, characterized in that hydroxyethylazolyl-oxime derivatives of the formula (I) in Claim 1 or their acid addition salts or metal salt complexes are mixed with extenders and/or surface-active agents.